Europäisches Patentamt

**European Patent Office**

Office européen des brevets

(11) Numéro de publication: **0 296 927**
**A1**

## (12) DEMANDE DE BREVET EUROPEEN

(21) Numéro de dépôt: **88401426.7**

(22) Date de dépôt: **10.06.88**

(51) Int. Cl.⁴: **A 61 B 5/02**

(30) Priorité: **11.06.87 FR 8708129**

(43) Date de publication de la demande:
**28.12.88 Bulletin 88/52**

(84) Etats contractants désignés:
**AT BE CH DE ES FR GB GR IT LI LU NL SE**

(71) Demandeur: **BERTIN & CIE**
**Zone Industrielle Boîte postale 3**
**F-78373 Plaisir Cédex (FR)**

(72) Inventeur: **Chiaffi, Michel**
**5 rue des petits champs**
**F-75001 Paris (FR)**

**Schlegel, Raymond**
**3 rue Emmanuel Chabrier**
**F-78330 Fontenay Le Fleury (FR)**

**Weber, Jean-Luc Michel**
**Domaine de Fongrave**
**F-13300 Salon de Provence (FR)**

**Celliere, Jean-Claude**
**10 résidence Valcros Rue de Valcros**
**F-13090 Aix-en-Provence (FR)**

(74) Mandataire: **Colas, Jean-Pierre et al**
**Cabinet de Boisse 37, avenue Franklin D. Roosevelt**
**F-75008 Paris (FR)**

(54) **Appareil ambulatoire pour l'enregistrement de données relatives à la tension artérielle.**

(57) L'invention est relative à un appareil ambulatoire pour mettre en mémoire des données relatives au fonctionnement du système cardiovasculaire.

Un boîtier (3) porté par le brassard gonflable (1) lui-même, contient des piles d'alimentation, un motocompresseur miniaturisé, des vannes, un microprocesseur et des mémoires RAM. Les données fournies par un capteur de pression et un microphone (4) sont numérisées et compressées pour être emmagasinées dans une ou plusieurs mémoires RAM, dont le contenu est ensuite transféré dans l'ordinateur d'un centre de soins. Avantageusement, la disparition des bruits de KOROTKOV à la fin de la montée ou de la descente de la pression commande le début et la fin de la mise en mémoire.

FIG.:1

## Description

### Appareil ambulatoire pour l'enregistrement de données relatives à la tension artérielle

La présente invention concerne un appareil ambulatoire pour l'enregistrement de données relatives à la tension artérielle.

La méthode la plus classique de mesure de la tension artérielle utilise l'appareillage de PACHON, qui comprend, d'une part, un brassard gonflable placé au niveau du tiers inférieur du biceps brachial, où l'artère humérale peut être comprimée contre la paroi de l'humérus et, d'autre part, un instrument de détection qui peut être soit acoustique, et constitué alors d'un stéthoscope ou d'un microphone placé en aval du brassard, soit apte à détecter les variations de pression du brassard (appareil de WAQUEZ).

Dans les deux cas, après que le patient a fléchi à demi le bras, on gonfle le brassard à une pression supérieure à la pression artérielle maximale estimée, dans la pratique, à 0,27 bar ,environ, puis on procède à un dégonflage lent du brassard, par mise à l'air libre, ce dégonflage durant une à deux minutes. Pendant le dégonflage, on détermine d'abord la pression systolique, assimilée à la pression maximale du réseau artériel. Le passage par la pression systolique se traduit, sur un détecteur acoustique, par l'apparition du bruit émis par l'écoulement sanguin qui recommence à passer dans l'artère, c'est-à-dire le "premier bruit de KOROTKOV". Sur un détecteur de pression du brassard, ce passage se traduit par des oscillations de pression, celles-ci passant de la pression propre du brassard à une pression supérieure à l'arrivée de i'influx sanguin.

Au cours du dégonflage, on détermine ensuite la pression diastolique, assimilée à la pression minimale du réseau artériel, et le passage de la pression du brassard par cette pression diastolique se traduit, sur un détecteur acoustique, par la disparition du bruit, c'est-à-dire le dernier bruit de KOROTKOV, et sur un détecteur de pression, par la disparition des oscillations.

Il existe des appareillages de mesure ambulatoires de la tension artérielle qui permettent au patient de contrôler et/ou d'enregistrer celle-ci tout en se déplaçant à volonté. Ces appareils comportent, outre le brassard déjà mentionné et un capteur de type acoustique, un module de compression d'air pour le brassard, avec les moyens nécessaires de commande des opérations de gonflage et dégonflage du brassard, de déclenchement et d'arrêt des mesures, ainsi que de traitement des signaux émis par le capteur pour en extraire notamment la pression systolique, la pression diastolique et la fréquence cardiaque, et pour enregistrer ces données, en vue de les transmettre à un centre de soins, et éventuellement également en vue de les afficher. Certains de ces appareils comportent, en outre, un dispositif qui permet de procéder aux déterminations de données ci-dessus par mesure de pression lorsque la méthode acoustique devient inopérante.

Il est reproché à ces appareils, outre leur prix élevé, leur fonctionnement bruyant, et le poids et l'encombrement du module de commande, d'alimentation et d'enregistrement, qui doit être porté à la ceinture ou en bandouillère. D'un autre côté, les données qui sont fournies au centre de soins peuvent être perturbées par des phénomènes extérieurs sans que le praticien puisse en être avisé, car il n'a pas les moyens de revenir aux signaux initiaux du capteur.

Le document WO-A-86.03114 décrit un appareil ambulatoire du type ci-dessus, dans lequel les signaux du capteur de pression et du capteur acoustique sont envoyés, après amplification, sous forme analogique à un enregistreur équipé d'une bande magnétique destinée à être transmise au centre de soins. Les signaux sont également traités pour en extraire des valeurs de pression systolique et diastolique notamment, et ces valeurs sont stockées sous forme numérique pour l'information du porteur. Elles peuvent être également transmises au centre de soins. Ce système est compliqué, encombrant et lourd, et, surtout, d'une part, il peut fournir des informations au porteur en temps réel, ce qu'il est recommandé d'éviter et, d'autre part, il est loin de permettre au centre de soins de récupérer l'intégralité des mesures effectuées par l'appareil : la transmission par l'intermédiaire d'une bande magnétique et sous forme analogique est très imparfaite, et le traitement préalable qui précède la mise en mémoire, sous forme numérique, des résultats de calcul, fait perdre lui-aussi une grande partie de l'information.

La présente invention a pour but premier de fournir un appareillage qui permette au praticien du centre de soins de récupérer l'intégralité des mesures effectuées par l'appareil et ainsi lui permettre d'effectuer en temps différé une étude plus fine et plus fiable des phénomènes, et de détecter les anomalies survenant au niveau des signaux provenant des capteurs. Un second but de l'invention est de fournir un appareillage qui soit à la fois moins cher, moins bruyant, moins encombrant et plus léger que les appareillages ambulatoires existants.

L'invention fournit en conséquence un appareil ambulatoire pour l'enregistrement de données relatives à la tension artérielle, comprenant une source autonome électrique , un brassard gonflable qui peut être sélectivement relié à une source autonome de gaz comprimé ou à l'atmosphère, un capteur acoustique et un capteur de pression, capables de fonctionner simultanément et reliés à des moyens électroniques d'enregistrement des informations provenant desdits capteurs vue de leur exploitation dans une station de traitement de données, cet appareil ayant pour particularité qu'il comprend des moyens pour échantillonner et mettre sous forme numérique les signaux émis par lesdits capteurs, et pour enregistrer l'intégralité des résultats de l'échantillonnage et les restituer ultérieurement à la station de traitement en vue de son exploitation différée pour reconstituer les mesures d'origine et en extraire des éléments de diagnostic.

Par rapport aux appareillages existants, on transfère donc les moyens de traitement de l'information depuis l'appareil ambulatoire jusqu'à la station de traitement, par exemple un centre de soins, et on prévoit en revanche, sur l'appareil ambulatoire, des moyens convenables, naturellement plus puissants, pour stocker , de préférence sous forme compactée ,toute l'information "brute", à savoir l'ensemble des mesures de pression et de bruit.

On observera que l'appareil, en fait, n'est aucunement un appareil de mesure de la tension artérielle, car il ne contient pas de moyens pour calculer cette tension à partir des signaux des capteurs, celle-ci n'est déterminée qu'a posteriori et par analyse des mesures dans la station de traitement de données.

Selon l'invention, l'appareil est donc essentiellement un enregistreur passif, seul le praticien du centre de soins ayant accès, grâce à un équipement informatique, à la totalité de l'information déductible de l'enregistrement des signaux des capteurs.

Sur un sujet normal, les indications données par les méthodes pressiométrique et acoustique sont globalement concordantes, et il y a donc redondance apparente à utiliser les deux méthodes simultanément; cependant, on doit se souvenir que l'appareil est précisément surtout destiné à détecter des anomalies du fonctionnement du système cardiovasculaire. D'autre part, un phénomène perturbateur non désiré, d'origine interne (musculaire par exemple), ou externe (mouvement du brassard) peut être éliminé par comparaison des signaux. Il est clair que de telles comparaisons ne sauraient être du ressort d'un ordinateur "embarqué". Il faut donc que les signaux initiaux soient transmis dans leur intégralité, ou presque, au praticien du centre de soins. Ainsi dans les cas litigieux, le praticien peut analyser lui-même les signaux et lever un doute éventuel.

Suivant une modalité avantageuse, l'appareil comprend des moyens pour limiter la vitesse de montée de la pression de gaz dans le brassard lorsque celui-ci est relié à la source de gaz comprimé, à une valeur suffisamment faible pour que plusieurs bruits de KOROTKOFF puissent être identifiés, et il est prévu des moyens pour commander le début d'un cycle de mesures après un temps prédéterminé suivant la disparition des bruits de KOROTKOFF identifiés pendant la phase de montée de la pression. De cette façon, on évite de gonfler le brassard plus que cela n'est nécessaire. De manière similaire, la disparition des bruits de KOROTKOFF en cours de dégonflage peut être utilisée pour mettre fin au cycle de mesures. On améliore ainsi, d'une part, le confort du porteur, et d'autre part, l'autonomie de l'appareil à poids égal, ou son poids à autonomie égale.

De préférence, l'appareil comprend des moyens pour faire subir aux signaux des capteurs, après leur échantillonnage pour mise sous forme numérique, un traitement de compression de données numériques avant leur stockage dans au moins une mémoire statique.

On limite ainsi considérablement l'importance des mémoires de l'appareil, sans altération appréciable du contenu des informations.

Dans ce cas, l'appareil comprend, de façon préférée :

- des moyens pour échantillonnner sur une première fréquence le signal analogique du capteur de pression et le numériser,
- des moyens pour comprimer les données "pression" ainsi numérisées par détermination des différences et des changements de signes entre valeurs numériques successives,
- une première mémoire RAM pour stocker les données comprimées du capteur de pression,
- des moyens pour échantillonner sur une seconde fréquence le signal analogique du capteur acoustique et le le numériser,
- des moyens pour comprimer les données acoustiques ainsi numérisées par comptage séparé des valeurs successives de niveau nul et détermination des valeurs correspondant aux pics ou seulement de la valeur maximale de chaque pic,
- une seconde mémoire RAM pour stocker les valeurs comprimées du capteur acoustique.

Par "première"et "seconde mémoire", le technicien comprendra qu'on peut entendre des première et seconde zones d'une même mémoire.

Une telle disposition permet de réaliser une partie électronique très compacte. On a pu établir que la capacité totale nécessaire des mémoires pouvait être de 64 kilo-octets.

Une cause de mesure incorrecte importante réside dans les liaisons entre le coffret, qui contient le module de commande et d'alimentation, et le brassard. Dans les systèmes actuels, ce coffret est porté à la ceinture ou en bandoulière, c'est-à-dire à distance du brassard. Des réductions importantes de poids et d'encombrement ont permis de fixer le coffret sur le brassard lui-même, d'où réduction importante des risques d'incidents, et en même temps amélioration du confort.

Ces réductions ont été rendues possibles par un certain nombre de modalités qu'on va maintenant indiquer, ou qui apparaîtront dans la description qui va suivre.

Les électrovannes sont constituées à partir de relais magnétiques bi-stables, dont la partie magnétique mobile est pourvue d'un revêtement d'élastomère qui, en position d'obturation, ferme un orifice de mise à l'air, et en position d'ouverture, découvre cet orifice.

De préférence, le moyen pour fournir un gaz comprimé est un microcompresseur à membrane, à faible poids et faible consommation.

Toutefois, dans certains cas, par exemple si l'entretien du compresseur est difficile, on peut prévoir que le gaz comprimé est fourni par une bouteille de gaz, munie d'une électrovanne de type classique et d'un détendeur. Cette solution aboutit cependant à des réalisations plus lourdes et plus encombrantes.

L'invention va maintenant être exposée de façon plus détaillée à l'aide d'un exemple pratique, non limitatif, illustré par les dessins. Parmi ceux-ci :

La figure 1 est une vue en perspective du dispositif monté sur le bras d'un sujet.

La figure 2 est une vue en plan du boîtier, couvercle enlevé.

La figure 3 montre un enregistrement type des données receuillies en fonction du temps :

la courbe A montre l'enregistrement des pressions, la courbe B, en unités différentes, la composante fluctuante de la pression, et la courbe C, la tension sortie du micro qui enregistre les bruits de KOROTKOV.

La figure 4 est un schéma de blocs de l'électronique.

La figure 5 est une schéma partiel montrant le rôle des électrovannnes.

La figure 6 est un graphique synoptique d'une mesure.

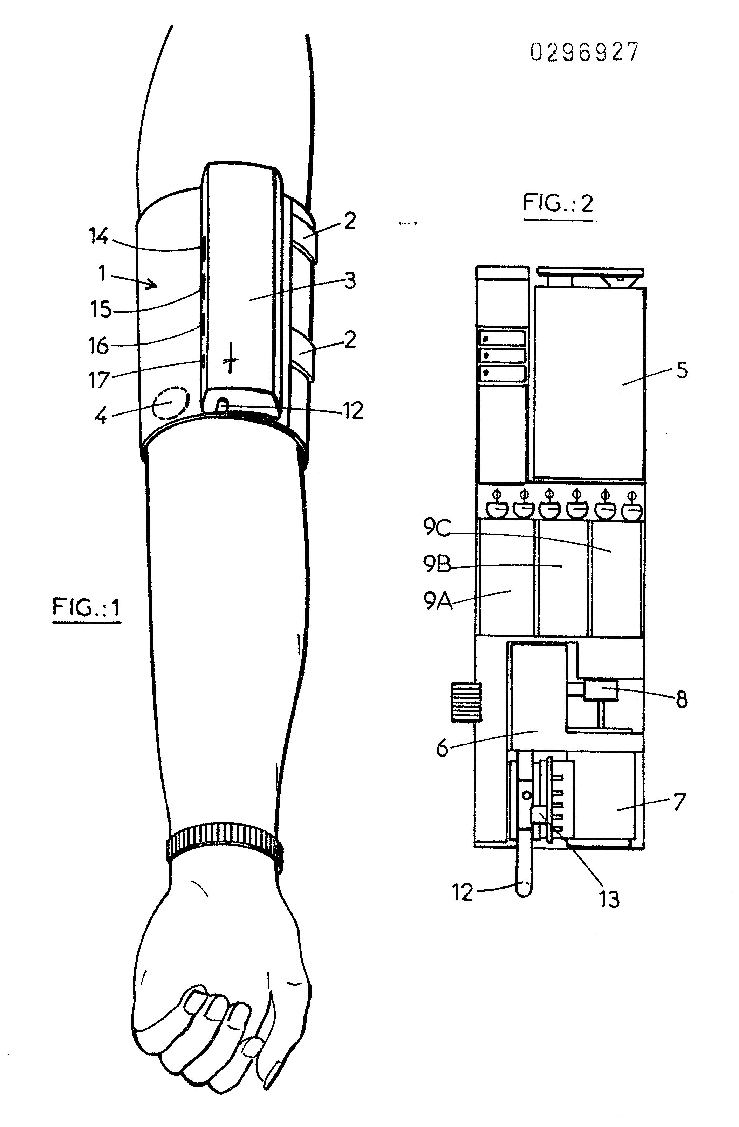

Le dispositif objet de la figure 1 se compose d'un brassard gonflable 1 de type classique, d'une contenance de 300 cm3, qui se fixe sur le bras du sujet à l'aide de deux bracelets 2, et qui porte, sur sa face externe, un boîtier 3, dont les dimensions, dans l'exemple décrit, sont 140 x 55 x 22 mm. Ce boîtier, sous ce volume réduit, contient l'ensemble de l'appareillage nécessaire, à l'exception d'un microphone 4, qui est placé dans le bras sard, en dessous de la partie gonflable.

L'appareillage comprend, pour son alimentation, une pile 5 alcaline de type courant Mn/ZnO de 9 volts nominaux, de capacité 500 milliampères-heure. On pourrait utiliser également des éléments au Li/MnO2, qui, pour un encombrement et un poids égal, procurent une capacité plus de deux fois supérieure, mais sont nettement plus coûteux.

Le moyen de fourniture de gaz comprimé est ,un groupe moto-compresseur d'air, comprenant un compresseur à membrane 6 du modèle "3003D" de la Société ASF, commandé par un moteur 7 du type 16M11 - 7,5 Vcc de la Société ESCAP par l'intermédiaire d'une transmission 8 par bielle et manivelle. Ce groupe est capable d'un débit, à pression nulle, de 2500 cm3/minute. Le temps de gonflage du brassard est d'environ 30 secondes. La consommation de courant, pour un gonflage, est inférieure à 0,5 milliampère-heure. Son carter a été entaillé pour gagner de la place.

Une autre solution pour la fourniture de gaz comprimé consiste dans l'utilisation d'un réservoir à gaz comprimé, muni d'une électrovanne et d'une soupape de sécurité. Parmi les gaz utilisables, le préféré est ledichlorodifluorométhane. Ce dernier présente l'avantage d'un point de liquéfaction à basse pression (quelques bars) à la température ambiante. Une capsule de 100 cm3 permet une autonomie suffisante pour 100 gonflages. Le poids de la capsule, en alliage d'aluminium, est de 30 g. On peut aussi utiliser le dioxyde de carbone, le bromodifluorométhane et le chloropentafluoroéthane. Tous ces gaz sont emmagasinés à l'état liquide dans le réservoir.

Le coffret d'appareillage contient encore des vannes de dégonflage 9A, 9B, 9C du brassard, qui envoient à l'atmosphère le gaz comprimé, de façon contrôlée, de manière à réduire la pression à une vitesse convenable, c'est-à-dire lente et régulière pendant la phase d'acquisition des données, rapide après la fin de cette phase.

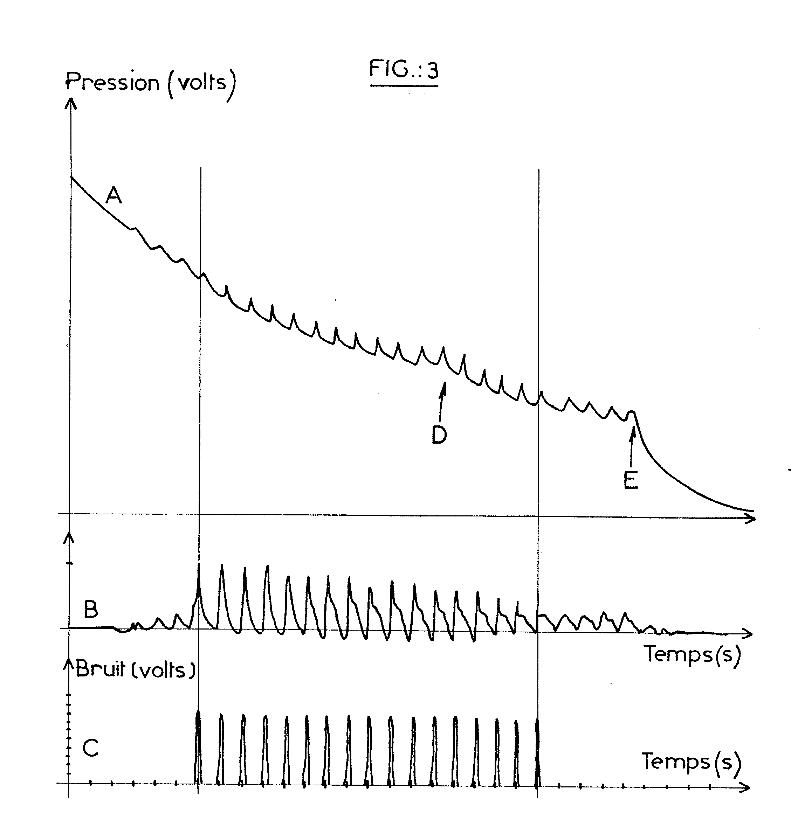

Plus exactement, le signal émis par le capteur de pression comprend une "composante continue" qui corres pond à la décroissance de la pression sous l'effet des vannes et une "composante fluctuante" qui correspond à l'effet des battements du coeur. Comme on le verra plus loin, il est préférable de séparer ces deux composantes. Pour cela, il est avantageux que la décroissance de la pression due aux vannes soit lente, surtout au voisinage des pressions systolique et diastolique. D'un autre côté, il convient de ne pas prolonger la durée totale d'une mesure au-delà du nécessaire. Il a été jugé que l'emploi de deux vannes montées en parallèle, et dont seule la première 9A est ouverte au début des mesures alors que l'autre 9B s'ouvre entre la pression systolique et diastolique, constitue un compromis acceptable. La troisième vanne 9C, quand à elle, s'ouvre une fois qu'on a passé la pression diastolique. Le résultat de cette méthode est visible à la figure 3, où les flèches D et E montrent l'instant de l'ouverture des vannes 9B et 9C.

Il a été constaté que les électrovannes disponibles dans le commerce avaient une consommation d'énergie très importante, supérieure à celle du micro-compresseur, si bien qu'il a été nécessaire de mettre au point des vannes susceptibles de fonctionnner avec une consommation d'énergie très faible.

La solution a été fournie par l'utilisation de relais magnétiques bistables modifiés par le dépôt, sur la partie mobile du relais, d'une garniture en matière élastomère. Un tube métallique est disposé de telle façon que, dans une position du relais, la garniture en matière élastomère vient obturer l'orifice de sortie du tube, alors que, dans l'autre position du relais, l'orifice est libéré.

Dans la pratique, on a utilisé des relais de la société "SDS", de tension nominale 5V, dont le basculement est déjà obtenu avec impulsion de tension de 4 volts pendant une durée de 5 millisecondes. il est possible de supprimer les contacts électriques qui sont inutiles dans l'utilisation comme vanne, mais on peut aussi les utiliser à d'autre fonctions, le relais continuant alors de fonc tionner comme relais électrique.

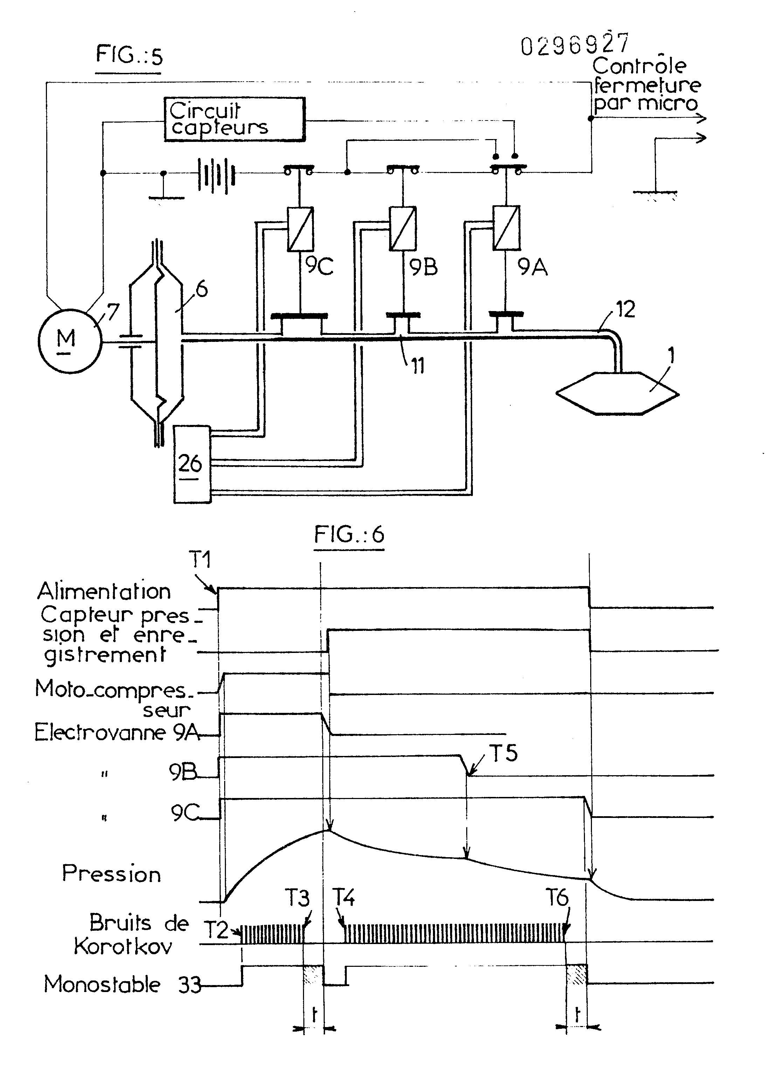

Plus précisément, ainsi que cela est représenté à la figure 5, le relais constituant la première vanne 9A de décharge lente commande, quand il passe en position d'ouverture de la vanne, l'arrêt du compresseur et la mise en route de l'alimentation des capteurs et de l'électronique de traitement initial de leurs signaux. Le relais constituant la vanne de décharge rapide 9C commande, de la même façon, l'arrêt de l'alimentation des capteurs et de l'électronique de traitement initial de leurs signaux. La fermeture simultanée des trois vannes 9A, 9B, 9C est une condition pour la mise en route du compresseur.

On notera que les vannes de l'invention conviennent pour tous les cas où on désire un fonctionnement sûr avec une consommation d'énergie très faible pour commander des débits de fluide ou des pressions relativement peu importants, par exemple dans les applications d'analyses, ou dans les industries de l'espace.

On notera également que les contacts qui ne servent pas pour la commande, au lieu de servir à la commande d'autres appareillages, peuvent servir seulement au contrôle de position des vannes.

Les vannes ainsi constituées sont, dans l'appareil décrit, au nombre de trois, mais il est possible d'en prévoir un nombre différent.

Les vannes sont reliées à un collecteur commun 11, lui-même relié d'une part au compresseur et, d'autre part, à un conduit 12 de raccordement au brassard. Sur le collecteur est placé le capteur de pression 13. Celui-ci est du type LX602GB commercialisé par la Société IC SENSOR, mis au point pour l'industrie automobile, à jauge piezorésistive montée en pont de Wheatstone, il est associé à un régulateur de tension.

L'autre capteur, à savoir le capteur acoustique 4, qui est fixe sur le brassard, comme on l'a dit, est un microphone piezo-électrique de la société SPENGLER, utilisé déjà pour les tensiomètres d'autocontrôle qui sont dans le commerce.

Le boîtier contient encore les circuits électroniques qui seront décrits plus loin, et sur sa face externe, un poussoir 14 non accessible facilement, commandable par une pointe de stylo, et qui commande le démarrage du cycle de mesures pour 24 heures, un interrupteur 15 permettant au patient d'arrêter ou de reprendre les opérations de gonflage du brassard et d'acquisition de mesures pendant le cycle, et un autre poussoir 16 qui lui permet de déclencher une mesure volontaire. Le boîtier porte en outre un diode lumineuse 17 qui constitue un témoin de fonctionnement.

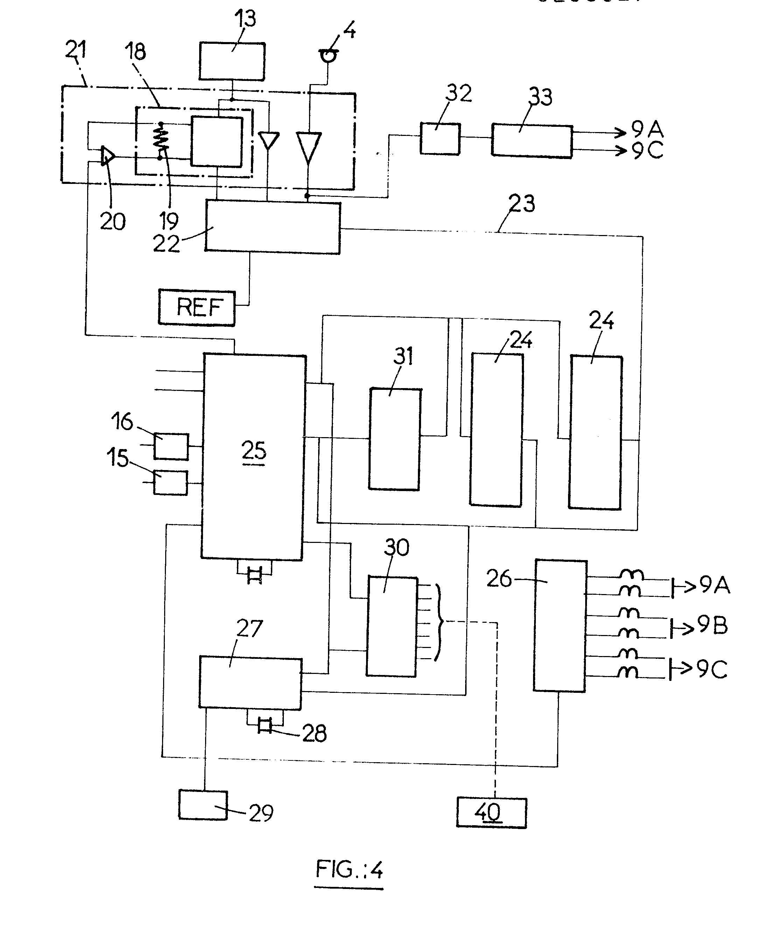

On va maintenant décrire la partie électronique correspondant aux traitement des signaux, dont un schéma d'ensemble est donné à la figure 4.

## I - Signal de pression

L'observation des signaux de pression, tels qu'on peut les voir à la courbe A de la figure 3, montre qu'il faut au moins 12 bits pour numériser correctement l'ensemble du signal de pression. Or, les convertisseurs 12 bits disponibles ont l'inconvénient de ne pas exister en CMS (montage de surface), d'être un peu délicats à mettre en oeuvre et surtout de ne pas avoir de multiplexeur intégré.

Pour résoudre cette difficulté, on a tiré parti du fait que le signal du capteur de pression peut s'analyser comme la somme d'une composante à variation lente, qui correspond à la décroissance de la pression commandée par les vannes, et d'une composante fluctuante, à fréquence assez régulière, qui est significative de la détection des impulsions provenant du système cardio-vasculaire. Pour numériser chacune de ces composantes séparément, il suffit d'un nombre de bits inférieur à celui qui est nécessaire pour traiter le signal de pression dans son ensemble.

On sépare donc les deux composantes par des moyens électroniques agissant en mode analogique, avant toute numérisation, par des moyens bien connus qui sont des filtres de fréquence passe-haut et passe-bas.

Un problème est alors apparu au sujet de la séparation de la composante à variation lente, qui est séparée par un filtre analogique 18 de constante de temps de 2 secondes : l'établissement de la tension moyenne aux bornes des condensateurs du filtre requiert une durée de 10 à 15 secondes, incompatible avec la durée totale de l'acquisition. Pour pallier cette difficulté, les résistances 19 du filtre 18 sont shuntées au moyen d'interrupteurs 20 analogiques pendant la durée de gonflage, ces interrupteurs sont commandés par un microprocesseur 25 décrit plus loin.

Il est cependant possible de ne pas faire appel à un filtre analogique "amont" pour séparer les deux composantes du signal de pression avant leur échantillonnage. On pourrait aussi bien utiliser en aval une technique de type informatique dite de "moyenne glissante", bien connue des praticiens, pour séparer les composantes des échantillons à variation lente et rapide.

La valeur maximale de la composante à variation lente doit être quantifiée en amplitude réelle lors de chaque cycle de mesures.

On notera qu'avec un échantillonnage sur 8 bits, soit 256 niveaux, si la pression maximale est de l'ordre de 250 millimètres de mercure, la précision sera de l'ordre du millimètre de mercure, soit 0,0013 bar. La fréquence des échantillonnages peut être très basse, par exemple une seconde, et il suffit d'enregistrer la diminution de la pression par rapport à la valeur maximale initiale, c'est dire que le volume d'information à enregistrer est très réduit.

La composante fluctuante (courbe B de la figure 3) n'exige pas une quantification précise en valeur absolue. En effet, lors du traitement ultérieur par le processeur du centre de soins, elle servira en premier lieu à déterminer le moment précis de la systole et celui de la diastole. Pour cela, on utilisera, de préférence, le point de pente maximale de l'enveloppe des maxima des pics de la composante fluctuante. En outre, l'examen de la forme des pics peut servir à éliminer des perturbations provenant de l'appareillage de mesure ou du mode opératoire, cependant, la hauteur absolue des pics n'a pas à être connue avec une grande précision. On dispose donc d'une certaine liberté pour amplifier le signal de composante fluctuante.

D'un autre côté, la connaissance de la forme de la composante fluctuante exige évidemment une fréquence d'échantillonnage bien plus élevée que celle de la composante à variation lente, si bien que le problème du stockage des informations est plus aigu. Dans un processus de compression ou compactage, dans lequel on ne met en mémoire que la différence entre deux valeurs du signal, successives, cette différence est d'autant plus faible que la fréquence d'échantillonnage est plus élevée, si bien qu'on peut affecter un plus petit nombre de bits à cette différence.

L'obtention du volume minimal d'information à mettre en mémoire constitue un problème d'optimisation où le gain de l'amplificateur et la fréquence d'échantillonage doivent être déterminés en fonction des caractéristiques du convertisseur analogique-numérique.

Dans la pratique, on a choisi un convertisseur du type ADC 829, de la Société NS, qui possède huit entrées analogiques, dont trois sont utilisées, et une sortie parallèle, et dont la tension de référence est de 2.85 V. Ce convertisseur comporte un multiplexeur intégré et un échantillonneur-bloqueur qui assure le maintien de la tension pendant la conversion.

Ce convertisseur traite à la fois la composante à variation lente et la composante fluctuante du signal de pression, et le signal de bruit.

Le gain de l'amplificateur et la fréquence d'échantillonnage de la composante fluctuante sont choisis de façon à optimiser le volume d'information à stocker.

La fréquence d'échantillonnage de composante fluctuante a été choisie égale à 20 Hz.

Le gain a été choisi de façon à ce que les différences successives qui sont à stocker présentent les caractéristiques suivantes :

- La majorité (de l'ordre de 80%) des valeurs peut être codée sur 4 bits pour la valeur absolue.

- Les valeurs non codables sur 4 bits le sont toutes sur 6 bits (ces valeurs correspondent à une montée rapide du signal pendant la phase d'éjection systolique).

- Les changements de signe de ces valeurs sont peu fréquents (2 par période cardiaque) et par conséquent seuls les changements de signe sont codés.

### Algorithme de stockage de la composante fluctuante:

a) deux codages distincts sont employés suivant les valeurs pour tenir compte des dynamiques différentes, à savoir :

valeur $\leq$ 14 stockage sur un quadruplet

14 < valeur < 63 stockage sur 7 bits d'un octet (y compris bit de signe), le bit restant servant d'indicateur pour le changement de code éventuel des valeurs suivantes.

Un octet particulier est réservé à l'identification du changement de code par exemple 1111 0000 ou 1111 1111 pour le passage de "valeurs $\leq$ 14" à "valeurs > 14".

b) du fait de la permanence assez longue du signe, seul le changement de signe est codé. Le quadruplet 1111 est réservé à cet usage.

Si on considère que le programme journalier de mesure comporte 100 mesures de 30 secondes chacune, le calcul montre que le volume de mémoire nécessaire pour conserver les signaux, après compression,, est au maximum de 40 kilo-octets.

### II - Signal microphonique (bruits de KOROTKOV)

Le signal du microphone présente des pics très étroits, séparés par des valeurs nulles. Ces valeurs nulles peuvent être forcées au moyen d'un léger offset négatif de l'ampli de sortie, ce qui est interprété comme une valeur nulle par le convertisseur analogique-numérique, elles représentent à peu près 85% des valeurs échantillonnées.

Un échantillonnage à 40 Hz suffit largement pour acquérir le signal, il permet d'obtenir 4 à 6 points représentatifs par pic.

Le gain de l'amplificateur des bruits de KOROTKOV est choisi de façon à ce que les amplitudes maximales correspondent à la pleine échelle du convertisseur.

Dans ces conditions, chaque pic est en moyenne déterminé par 8 valeurs.

On stocke seulement les valeurs correspondant aux pics. Compte tenu de la forme du signal, une quantification sur 8 bits est largement suffisante.

### Algorithme de stockage des signaux de microphone.

Chaque point significatif est stocké sur 1 octet (la dynamique du signal étant importante, les différences ne tiennent pas sur un quadruplet). Entre chaque point significatif, on stocke le nombre de valeurs nulles successives. Afin de ne pas alourdir (introduction de code de changement de codage), on convient d'employer des octets pairs pour stocker le nombre de 0, ce qui permet de décompter jusqu'à 127 valeurs nulles successives (11111110). L'identification de stockage de 0 est reconnue par la mise à "0" du bit de poids faible.

Le nombre de 0 est égal à la valeur de l'octet divisé par 2.

Pour identifier les valeurs significatives (non nulles), on convient d'arrondir si nécessaire à l'octet impair immédiatement supérieur, ce qui n'introduit qu'une faible erreur absolue.

Si on considère qu'une mesure de tension de 30 secondes détecte au maximum 25 pics de bruit de KOROTKOV, on aboutit par enregistrement à 200 octets pour les pics et 26 octets pour les interpics, soit, pour les 100 mesures journalières, à un maximum de 226 x 100 = 22,6 kilo-octets.

En ajoutant ce nombre au maximum de 40 kilo-octets correspondant aux mesures de pression, on voit qu'on reste au-dessous des 64 kilo-octets. Ce stockage est fait à l'aide de deux mémoires RAM statiques CMOS de 32 kilo-octets.

Sur la figure 4, l'ensemble des moyens de traitement analogique est groupé dans un bloc 21, d'où sortent :

- la composante continue J1 du signal de pression,

- la composante fluctuante J2 du signal de pression,

- le signal du micro J3,

ces trois signaux étant dûment filtrés et amplifiés.

Ces signaux sont envoyés au convertisseur analogique-numérique 22 puis un bus 23 achemine les signaux, numérisés et comprimés, vers deux mémoires RAM 24 en parallèle, chacun de 32 kilo-octets. Il s'agit de boîtiers CMS de la société NEC, de technique CMOS statique.

Un microprocesseur 25 constitue l'organe central de commande du dispositif.

On notera qu'il s'agit en fait d'un séquenceur. Il n'est pas conçu en effet pour interpréter les informations issues des capteurs, hormis la disparition des bruits de KOROTKOFF ayant valeur d'ordre, mais pour émettre des signaux de commande dans un certain ordre au cours d'un cycle de mesures répété à des instants prévus à l'avance. Il comporte un mode veille et un mode sommeil. C'est ce dernier qui est utilisé entre les mesures.

Il commande l'ouverture des vannes à travers un interface 26, la mise en action des moyens de traitement des signaux de pression, comme on l'a vu, ainsi que le remplissage des mémoires et l'envoi de leur contenu sur un interface 30 de liaison avec l'ordinateur 40 du centre de traitement.

Lors de la mise en route du compresseur, commandée par un signal émis par le microprocesseur à travers un circuit logique qui n'assure cette mise en route que si les électrovannes sont fermées, les circuits des capteurs 4, 13 sont également fermés. Les signaux correspondants sont convoyés au microprocesseur, après passage dans le convertisseur, bien entendu.

On va décrire maintenant une particularité des moyens de commande du compresseur et de certaines électro vannes. Les signaux J3 du microphone 4, après traitement analogique, sont envoyés, après mise en forme dans un circuit approprié 32, sur une bascule monostable redéclenchable 33, ayant une constante de temps t de 3 secondes. Cettebascule commande l'électrovanne 9A, qui, agissant en relais électrique, commande à son tour le moteur 7 du compresseur.

Pendant la phase de montée de la pression, c'est-à-dire du gonflage du brassard, les bruits de KOROTKOV apparaissent pour une pression voisine de la pression diastolique, puis disparaissent lorsqu'on a dépassé la pression systolique. Le premier bruit de KOROTKOV détecté active la bascule monostable 33.

Attendu que la constante de temps propre de la bascule, 3 secondes, est largement supérieure au temps qui s'écoule entre deux bruits de KOROTKOV, la bascule reste activée jusqu'à la fin du temps pendant lequel les bruits de KOROTKOV sont perceptibles, et ne change d'état que trois secondes après le dernier de ces bruits. Elle commande alors l'ouverture de l'électrovanne 9A et l'arrêt de compresseur, ainsi que le début d'une phase de mesure.

Un processus analogue commande l'ouverture de la vanne 9C en fin de prise tensionnelle.

En variante, la présence des bruits de KOROTKOV peut être détectée par le microprocesseur qui actionne directement l'ouverture des vannes après une temporisation sur la disparition du signal.

Ainsi, la fin de gonflage est commandée par la disparition des bruits de KOROTKOV, c'est-à-dire que la pression maximale est supérieure à la pression systolique d'une quantité sensiblement constante. La durée du gonflage est ainsi réduite au minimum nécessaire pour une mesure sûre. Dans l'art antérieur, la pression maximale est, en générai, fixée à l'avance, ce qui entraîne une gêne non nécessaire pour le patient. Cela est encore plus sensible dans le cas de l'utilisation d'un microcompresseur, qui a une durée de gonflage assez grande, et peut difficilement être totalement insonorisé dans un appareil portable.

On notera que l'équipement électronique, en dehors de l'horloge incluse dans le microprocesseur 25, comporte une autre horloge 27 pour déclencher les acquisitions de données et enregistrer l'heure, dans le but de corréler les mesures avec l'activité du sujet, surtour en cas de déclenchement de mesure volontaire. Cette horloge est équipée d'un quartz de montre 28 et d'une pile de sauvegarde 29.

Dans le pratique, le microprocesseur est du type 87C51 ou 80C51 de la société INTEL et l'horloge est un circuit intégré CMS.

Le mode opératoire est facile à comprendre compte tenu de ce qui précède, il est illustré à la figure 6.

Au centre de soins, le praticien fixe l'horaire des mesures de la journée, et l'entre dans le microprocesseur 25, il met à l'heure l'horloge 27, si cela est nécessaire.

Pendant la journée, le microprocesseur déclenche une mesure, soit conformément à l'horaire établi, soit sur une impulsion reçue du poussoir 16 quand le sujet désire une mesure volontaire.

Description d'un cycle de mesure.

A un instant prédéterminé T1, l'horloge de séquencement des prises tensionnelles délivre un signal qui met le microprocesseur 25 en mode opératoire et lui donne le contrôle.

Celui-ci déclenche la fermeture des trois vannes 9A, 9B, 9C. La vanne 9A, fonctionnant en relais, autorise l'alimentation du compresseur 6 et la pression monte dans le brassard.

La vanne 9C fonctionnant également en relais, permet, en position fermée, l'alimentation du circuit de mesure.

La durée de gonflage est pilotée par la présence des bruits de KOROTKOV (apparition à l'instant T2). Le fonctionnement du compresseur est interrompu après un délai fixé t après la disparition des bruits à l'instant T3. (Dans une variante, le fonctionnement du compresseur est interrompu lorsque la pression a dépassé d'une valeur fixée la pression atteinte lors de la disparition des bruits).

L'arrêt du compresseur est commandé par l'électrovanne 9A fonctionnant en relais. Simultanément à l'arrêt du compresseur, l'électrovanne 9A amorce la mise à l'atmosphère du circuit à faible débit. Simultanément à la commande d'électrovanne, le microprocesseur 25 déclenche le processus de mesure et d'acquisition (instant T3 + t).

A un instant T4 les bruits de KOROTKOV apparaissent.

A un instant T5, la seconde électrovanne 9B s'ouvre pour accélérer la mise à l'atmosphère. Cette ouverture est commandée par le microprocesseur lorsque la composante à variation lente de la pression passe au-dessous d'une valeur qui est une fraction prédéterminée de la pression à l'instant T3 + t du début des mesures.

A un instant T6, les bruits de KOROTKOV disparaissent, ce qui déclenche à l'instant T6 + t l'ouverture de l'électrovanne 9C de décompression rapide. Cette même électrovanne agissant en relais ouvre le circuit d'alimentation des capteurs et de conditionnement analogique.

Après un temps prédéterminé, le microprocesseur se met en état de sommeil en attente de la prochaine impulsion d'horloge.

Après une période de 24 heures environ, (par exemple), le boîtier est connecté sur l'ordinateur 40 du centre de soins, soit par l'intermédiaire d'un système de télécommunications, soit directement si le sujet retourne au autre soins ou y fait parvenir l'appareil. L'ordinateur 40 commande, par l'intermédiaire du microprocesseur 25, le transfert du contenu des mémoires 24 dans les mémoires du centre de soins et leur remise à zéro. Les piles 5 et 29 sont contrôlées et changées si nécessaire.

**Revendications**

1. Appareil ambulatoire pourl'enregistrement de données relatives à la tension artérielle, comprenant une source autonome d'énergie électrique, un brassard gonflable (1) qui peut être sélectivement relié à une source autonome de gaz comprimé ou à l'atmosphère, un capteur acoustique (4) et un capteur de pression (13), capables de fonctionner simultanément et reliés à des moyens électroniques d'enregistrement des informations provenant desdits capteurs en vue de leur exploitationdans une station (40) de traitement de données, caractérisé en ce qu'il comprend des moyens pour échantillonner et mettre sous forme numérique les signaux émis par lesdits capteurs (4, 13), et pour enregistrer l'intégralité des résultats de l'échantillonnage et les restituer ultérieurement à la station de traitement en vue de leur exploitation différée pour reconstituer les signaux d'origine et en extraire des éléments de diagnostic.

2. Appareil selon la revendication 1, caractérisé en ce qu'il comprend des moyens pour limiter la vitesse de montée de la pression de gaz dans le brassard, lorsque celui-ci est relié à la source de gaz comprimé, à une valeur suffisamment faible pour que plusieurs bruits de KOROTKOFF puissent être identifiés, et en ce qu'il est prévu des moyens pour commander le début d'un cycle de mesures après un temps prédéterminé suivant la disparition des bruits de KOROTKOFF identifiés pendant la phase de montée de la pression.

3. Appareil selon la revendication 1, caractérisé en ce qu'il est prévu des moyens pour commander la fin d'un cycle de mesures après un temps prédéterminé suivant la disparition des bruits de KOROTKOFF identifiés pendant la phase de dégonflage du brassard.

4. Appareil selon la revendication 2, caractérisé en ce que lesdits moyens pour commander le début et/ou la fin d'un cycle de mesures comprennent une bascule monostable redéclenchable (33) dont la constante de temps est supérieure à la durée qui sépare deux bruits de KOROTKOV consécutifs.

5. Appareil selon l'une des revendications 1 à 4, caractérisé en ce qu'il comprend des moyens (22) pour faire subir aux signaux des capteurs après leur échantillonnage et mise sous forme numérique un traitement de compression de données numériques, avant leur transfert dans au moins une mémoire statique (24).

6. Appareil selon une des revendications 1 à 5, caractérisé en ce qu'il comprend des moyens (19) pour extraire des signaux du capteur de pression (13) une composante à variation lente et une composante fluctuante, et pour numériser et comprimer ces deux composantes de manière indépendante.

7. Appareil selon la revendication 6, caractérisée en ce que les moyens pour extraire la composante à variation lente comprennent un filtre analogique amont (18), dont au moins une résistance (19) est mise en court-circuit pendant que le brassard est relié à la source de gaz comprimé.

8. Appareil selon la revendication 6, caractérisé en ce que les moyens pour extraire la composante fluctuante comprennent un circuit informatique aval opérant suivant une technique de "moyenne glissante".

9. Appareil selon l'une des revendications 1 à 8, dans lequel le brassard est relié à l'atmosphère par l'intermédiaire d'électrovannes commandées, caractérisé en ce que celles-ci sont constituées par des relais électromagnétiques bistables (9A à 9C) dont l'armature mobile, constituant un clapet, comporte une partie revêtue d'une matière élatomère.

10. Appareil selon la revendication 9, caractérisé en ce qu'au moins une desdites électrovannes (9A à 9C) fonctionne également comme relais électrique.

11. Appareil selon la revendication 9, caractérisé en ce que la première électrovanne (9A) qui s'ouvre pendant la phase de gonflage agit également comme relais pour interrompre la liaison entre le brassard et la source de gaz comprimé.

12. Appareil selon l'une des revendications 1 à 11, caractérisé en ce qu'il comprend un séquenceur (25) qui commande, en fonction d'un programme, la mise en action des différents organes de l'appareil au cours d'un cycle de mesure, ainsi que son propre passage du mode "veille" au mode "repos" à la fin de ce cycle.

13. Appareil selon l'une des revendications 1 à 12, caractérisé en ce que la source autonome de gaz sous pression est un mirocompresseur alimenté par une pile autonome.

14. Appareil selon l'une des revendications 1 à 12, caractérisé en ce que la source autonome de gaz sous pression est une bouteille de gaz comprimé.

15. Appareil selon l'une des revendications 1 à 14, caractérisé en ce qu'il comprend un boîtier (3) contenant la source autonome d'énergie (5), la source autonome de gaz comprimé (6, 7) et les moyens électroniques de commande, de traitement et d'enregistrement, ce boîtier étant de dimensions et poids suffisamment réduits pour pouvoir être fixé sur le brassard (1) lui-même, l'ensemble pouvant être porté sous les vêtements du sujet.

0296927

FIG.:2

FIG.:1

0296927

FIG.:3

Pression (volts)

A

D

E

B

Temps(s)

Bruit (volts)

C

Temps(s)

FIG.:4

FIG.:5

0296927

Circuit capteurs

Contrôle fermeture par micro

9C  9B  9A

6  7  M  12  1  11  26

FIG.:6

T1

Alimentation

Capteur pression et enregistrement

Moto_compresseur

Electrovanne 9A

"  9B  T5

"  9C

Pression

Bruits de Korotkov  T2  T3  T4  T6

Monostable 33

t  t

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,X | WO-A-8 603 114 (UNIVERSITY OF NORTH CAROLINA AT CHAPEL HILL) * Résumé; page 13, lignes 14-24; page 14, lignes 13-28; figures 1-3 * | 1,5,12-14 | A 61 B 5/02 |
| D,A | | 2,6,7,9 | |
| A | MEDICAL & BIOLOGICAL ENGINEERING & COMPUTING, vol. 23, no. 5, septembre 1985, pages 459-465, Stevenage, GB; K. YAMAKOSHI et al.: "Long-term ambulatory monitoring of indirect arterial blood pressure using a volume-oscillometric method" * Page 459, paragraphe 2: "Materials and methods"; figures 1-3 * | 1,2,5,9,11-13,15 | |
| A | GB-A-2 141 344 (MATSUSHITA ELECTRIC WORKS LTD) * Résumé; figures 1,4 * | 15 | |
| A | US-A-3 978 848 (D.H. YEN et al.) * Colonne 2, ligne 56 - colonne 3, ligne 20 * | 6,7 | DOMAINES TECHNIQUES RECHERCHES (Int. Cl.4)  A 61 B |
| A | US-A-4 671 290 (S. MILLER et al.) * Résumé; colonne 3, lignes 21-68; figure 6 * | 8 | |

Le présent rapport a été établi pour toutes les revendications

| Lieu de la recherche | Date d'achèvement de la recherche | Examinateur |
|---|---|---|
| LA HAYE | 13-09-1988 | HUNT,B.W. |